(19) Europäisches Patentamt
European Patent Office
Office européen des brevets

(11) Numéro de publication: **0 236 151 A1**

(12) # DEMANDE DE BREVET EUROPEEN

(21) Numéro de dépôt: **87400069.8**

(22) Date de dépôt: **14.01.87**

(51) Int. Cl.⁴: **C 07 C 131/08**, C 07 D 213/30, A 61 K 31/15, A 61 K 31/44

(30) Priorité: **29.01.86 FR 8601223**

(43) Date de publication de la demande: **09.09.87 Bulletin 87/37**

(84) Etats contractants désignés: **AT BE CH DE ES FR GB GR IT LI LU NL SE**

(71) Demandeur: **PANMEDICA S.A., 1ère avenue-2065 M - L.I.D., F-06516 Carros (FR)**

(72) Inventeur: **Laruelle, Claude, Avenue Bellevue, F-06270 Villeneuve Loubet (FR)**
Inventeur: **Lepant, Marcel, 7 rue Mellarède, F-06100 Nice (FR)**
Inventeur: **Raynier, Bernard, 9 chemin du Malvan, F-06800 Cagnes (FR)**

(74) Mandataire: **Orès, Bernard et al, Cabinet ORES 6, Avenue de Messine, F-75008 Paris (FR)**

(54) **Dioximes O-substituées symétriques de beta-dicéto-cycloalkylènes benzo-condensés, leurs procédés de préparation et leur application comme médicaments.**

(57) La présente invention est relative à des dérivés de dioximes O-substituées symétriques de béta-dicéto-cycloalkylènes benzo-condensés.

Ces dérivés qui répondent à la formule générale I ci-après, constituent des médicaments à actions analgésique et anticonvulsivante remarquables.

Ils constituent également des inhibiteurs de l'agrégation plaquettaire.

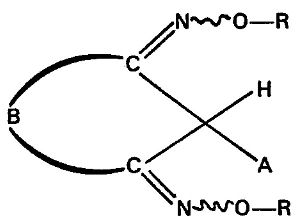

(I)

EP 0 236 151 A1

La présente invention est relative à des nouveaux dérivés β bis(alkyloxyimino)cycloalkylène symétriques et dont les deux doubles liaisons imines sont conjuguées à la ou aux doubles liaisons cycliques appartenant chacune à un cycle benzénique condensé.

Ils répondent à la formule générale suivante :

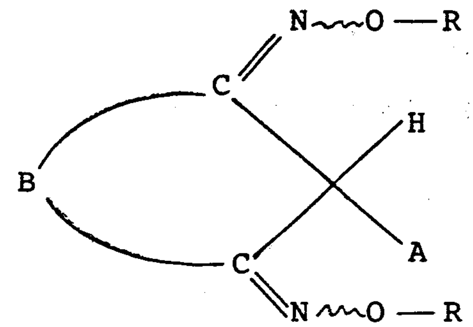

(I)

dans laquelle B représente le motif suivant :

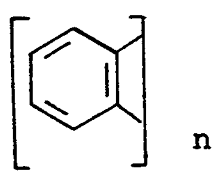

et où n = 1 ou 2.

Ainsi si B détermine une double liaison d'un cycle benzénique condensé, dans le cas où n = 1, on obtient bien un système de trois doubles liaisons conjuguées (N=C-C=C-C=N)

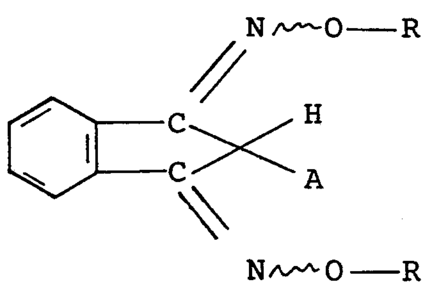

B pourra également déterminer un ensemble de 2 doubles liaisons de cycles benzéniques condensés conjuguées entre elles et avec les radicaux imines, on obtiendra dans ce cas un ensemble de quatre doubles liaisons conjuguées (N=C-C=C-C=C-C=N).

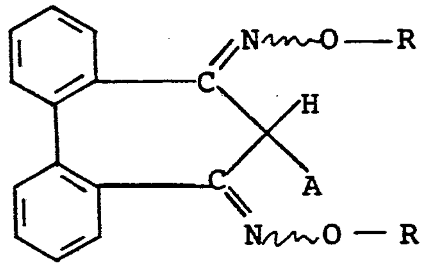

On voit donc que le cycle cycloalkylène comportera cinq ou sept atomes de carbone.

Dans le premier cas les deux cycles condensés détermineront un motif indane, dans le second les trois cycles condensés représenteront un dihydro-6,7 (5H) dibenzo [a,c] cycloheptène.

Les dérivés du type phényl-2 indane dione 1-3 sont connus depuis longtemps et utilisés en médecine humaine comme anticoagulants (phénindione), les homologues substitués sur le phényl-2 ont été étudiés un peu plus récemment et constituent un progrès dans la thérapeutique des anticoagulants (Fluindione).

Seules les oximes non substituées ont été synthétisées principalement à des fins analytiques, et leurs propriétés pharmacologiques n'ont pas suscité d'intérêt particulier.

D'autre part les systèmes tricycliques du type dibenzo [a,d] cycloheptène ont été très étudiés suite à la découverte de l'amitriptyline et de ses propriétés thérapeutiques dans la dépression.

Ainsi, le brevet U.S. 3.270.055 de H. ENGELHARDT et al. décrit des dérivés obtenus par une dialcoyl-amino alcoylation de l'oxygène de l'oxime des dérivés des dihydro-10, 11 dibenzo [a,d] cycloheptène-5 substitués éventuellement en 3.

Le brevet U.S. 3.349.128 (Colgate-Palmolive) décrit également des dérivés obtenus par une dialcoylamino-alcoylation des oximes dérivées des dibenzo [a,d] cycloheptènone-5 substituées éventuellement dans leurs

noyaux phényles.

Ces dérivés d'oximes peuvent exister sous la forme d'isomères syn ou anti et posséderaient des propriétés thérapeutiques en tant que sédatifs, antidépresseurs et relaxants musculaires.

Les systèmes tricycliques du type dibenzo [a,c] cycloheptène ont fait l'objet de quelques travaux antérieurs.

GÖDECKE (E.P. 112.584) décrit les dérivés (amino-alkyl)-7 ou (aminoalkoxy)-7 de la (5H)-dibenzo [a,c] cycloheptènone-5 comme agents thérapeutiques des maladies psychiques et des ulcères de l'estomac ou de l'intestin.

Dans Chem. Ber. 104 (1971) 1573, RIED et CONTE décrivent la synthèse du dioxo-5,7 dihydro-6,7 (5H)-dibenzo [a,c] cycloheptène et de sa dioxime non substituée.

La demanderesse a trouvé de façon surprenante que les bis-O alkyloximes de bêta dicétones de cycloalcènes à doubles liaisons entièrement conjuguées et de plus engagées dans des cycles benzéniques condensés présentaient des propriétés pharmacologiques inattendues.

La présente invention est relative à des nouveaux dérivés β di alkyloxyimino cycloalkylènes symmétriques et dont les doubles liaisons imine sont conjuguées à la ou aux doubles liaisons cycliques appartenant à un cycle ou à des cycles benzéniques condensés.

Ils répondent à la formule générale (I) suivante :

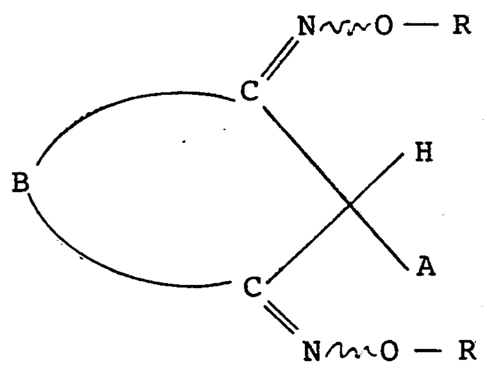

(I)

dans laquelle ;

- A représente l'hydrogène, un groupe phényle, ou un groupe phényle substitué par ;
  - un ou deux atomes d'halogène comme le fluor, le chlore, le brome, l'iode,
  - un ou deux radicaux alkyle inférieur qui peuvent être un groupement méthyle, éthyle, propyle, isopropyle, cyclopropyle, n-butyle, sec-butyle, tertio-butyle,
  - un ou deux radicaux alkoxy choisi parmi les groupes méthoxy, éthoxy, propoxy, isopropoxy, butoxy, tertiobutoxy
  - un ou des substituants choisis parmi les groupes ; nitro, cyano, diméthylamino, diéthylamino,
  - plusieurs substituants choisis dans chacun des différents groupes ci-dessus,
  - un groupement o-méthylènedioxy, o-éthylène dioxy,
- R représente :
  - un radical alkyle saturé ou insaturé, linéaire ou ramifié comprenant de 1 à 16 atomes de carbone,
  - un radical cycloalkylalkyle ou cycloalkyle comprenant au total de 4 à 16 atomes de carbone et dont le cycle comprend de 3 à 8 atomes de carbone,
  - un radical phényle ou phénylalkyle, le motif alkyle comprenant de 1 à 3 enchaînements méthylène,
  - un radical alkyle linéaire ou ramifié comprenant de 1 à 6 atomes de carbone substitué par un groupe -N $R_1R_2$, où $R_1R_2$ identiques ou différents sont des atomes d'hydrogène, des groupes alkyles linéaires ou ramifiés comprenant de 1 à 4 atomes de carbone éventuellement substitués par un groupe hydroxy, méthoxy, éthoxy, ou bien $R_1R_2$ identiques ou différents peuvent déterminer un hétérocycle azoté saturé ou insaturé, ou un hétérocycle saturé ou insaturé comprenant un atome d'azote et un hétéroatome qui peut être le soufre, l'oxygène ou l'azote, éventuellement substitué,

- un hétérocycle comprenant de 5 à 7 chaînons et comprenant 1 ou 2 hétéroatomes choisis parmi l'azote, le soufre, l'oxygène, relié par un enchaînement méthylène comprenant de 1 à 4 atomes de carbone,
- un radical alkyle linéaire ou ramifié saturé ou insaturé comprenant de 1 à 16 atomes de carbone et substitué par :
  . un groupement CN ou bien
  . un groupement -COOQ, Q étant l'hydrogène, un radical alkyle linéaire ou ramifié comprenant de 1 à 6 atomes de carbone, un radical phénylméthyle, phényle, ces cycles phényles étant éventuellement substitués par un halogène, un groupe cyano, un groupe méthyle, un groupe méthoxy ou un groupe trifluorométhyle.
- B représente l'enchaînement :

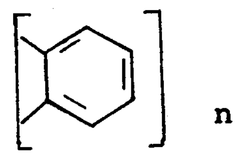

n étant égal à 1 ou 2,
- une double liaison d'un noyau benzénique condensé,
- deux doubles liaisons conjuguées entre elles et avec les groupes imino, comprises chacune dans un noyau benzénique condensé,

et leurs sels pharmacologiquement acceptables lorsque R contient un groupe salifiable.

B déterminera donc dans la formule générale I, pour n = 1 une β dioxime O-substituée de l'indane dione 1-3, pour n = 2 une β dioxime O-substituée du dioxo-5,7 dibenzo (a,c) cycloheptène.

La présente invention concerne l'ensemble des isomères géométriques ZZ,EE, ZE (Z signifiant syn et E signifiant anti) engendrés par les enchaînements C=N-O-R ou l'un quelconque des isomères obtenus à l'état pur par un procédé habituel de séparation comme la chromatographie sur colonne à partir du mélange des isomères.

Les dérivés selon la formule générale (I) présentent d'intéressantes propriétés médicamenteuses. Leurs activités pharmacologiques sont mises en évidence à l'aide des tests standards suivants :

- Activité anticonvulsivante chez la souris.

Une heure après administration du produit à tester, par voie orale à la souris, on pratique une injection IV de 50 mg/kg de pentaméthylène tétrazole.

La diminution ou la suppression des extensions toniques de l'animal mesurent l'activité anticonvulsivante. Les ED 50 des dérivés selon l'invention (dose efficace réduisant de 50 % les convulsions) varient de 200 à 5 mg/kg.

- Activité analgésique chez le rat.

Le writhing test est pratiqué chez le rat selon Koster (Fed. Proc. 18, 412, (1959)). Administrés à 100 mg/kg, certains dérivés selon la présente invention présentent une bonne activité analgésique.

- Effets antagonistes sur l'intestin isolé de cobaye.

Dans le text décrit par MAGNUS (Arch. F.D. Ges. Physiol. 102-123), le milieu de perfusion contenant 10 µg de produit/ml, les dérivés selon l'invention se révèlent antagonisés par la sérotonine, l'acétylcholine, l'histamine et le chlorure de baryum.

Pour certains dérivés notamment ceux décrits dans les exemples 14,15,16,17,18,19,22 , cet effet inhibiteur est encore sensible à une concentration égale à 0,1 µg/ml.

- Inhibition de l'agrégation plaquettaire in vitro.

L'inhibition de l'agrégation plaquettaire est mesurée in vitro selon BORN (Nature 4832, 927 (1962)) par induction au collagène ou à l'ADP.

Les dérivés selon la présente demande testés à une concentration de 100 µg/ml sont comparables voire supérieurs à l'aspirine, pris comme témoin, notamment

pour les produits décrits dans les exemples : 15,16, 17,18,19,20,21,24,25,26,27,28,29,30,32,33,34,35. L'induction de l'agrégation peut être également réalisée in vitro, par la sérotonine, l'acide arachidonique, la norépinéphrine. D'autre part, cet effet inhibiteur a été noté sur le plasma enrichi en plaquettes (PRP) d'origine animale et d'origine humaine.

- Activité ex vivo sur la coagulation et sur l'agrégation plaquettaire induite à l'ADP et au collagène chez le rat, après 6 jours d'administration.

L'ensemble des produits s'est révélé actif sur ce test, avec toutefois une intensité et une durée d'action (1/2 vie différente) variables.

- Allongement du temps de saignement.

L'étude est réalisée in vitro sur le rat et démontre un allongement significatif du temps de saignement après administration unique par voie orale des dérivés selon l'invention.

- Activité thrombolytique vis à vis de thrombi expérimentaux artériel ou veineux après administration per os chez le rat.

- Activité antiinflammatoire sur l'oedème de la patte du rat déclenché à la carragénine.

Les activités des dérivés selon l'invention sont variables mais beaucoup d'entre elles sont comparables voire supérieures à l'activité induite par l'aspirine.

- Tests généraux sur l'hémostase pratiqués in vitro comprenant le temps de céphaline kaolin (concerne les facteurs I, II, V, VIII, IX, X, XI, XII).

- Le temps de thrombine mesurant la transformation de fibrinogène en fibrine. La réponse à ces tests est excellente pour l'ensemble des dérivés de l'invention.

La transformation des dérivés de formule (I) dans laquelle R représente un radical hydrocarboné substitué par un groupement basique, en sels pharmacologiquement

acceptables, est effectuée par neutralisation avec un acide organique ou inorganique comme par exemple l'acide chlorhydrique, sulfurique, bromhydrique, phosphorique, méthane sulfonique, hydroxyéthane sulfonique, acétique, propionique , fumarique, oxalique, malique, lactique, citrique, tartrique, ascorbique ,maléique ,succinique, aspartique, glutamique, acétylaminoacétique, acétylaspartique, pyroglutamique.

La transformation des dérivés de formule I dans laquelle R représente un radical hydrocarboné substitué par un groupe carboxylique en sels pharmacologiquement acceptables est effectuée par neutralisation avec une base organique ou inorganique comme par exemple la soude, la potasse, l'ammoniaque, la magnésie, l'hydroxyde de calcium, la triéthylamine, l'isopropylamine, l'éthanol amine, le diéthylaminoéthanol, le diméthylaminoéthanol, la pipérazine, la N méthyl pipérazine, la morpholine.

Les compositions pharmaceutiques peuvent être mises sous des formes convenables pour une administration orale (par exemple comprimés, pilules, dragées, capsules, sirop) ou parentérale (par exemple solutions injectables). Des substances inertes peuvent être additionnées, telles que amidon, lactose, mannite, éthyl cellulose, talc, agent de dispersion ou agent de compression.

Cette addition se fait selon les méthodes de l'industrie pharmaceutique connues en elle-mêmes.

Une autre caractéristique de la présente invention est l'utilisation des dérivés de formule I et de leurs sels d'addition en thérapie humaine ou vétérinaire dans le traitement des affections résultant d'un dérèglement du fonctionnement plaquettaire et de l'agrégation sanguine des affections du système nerveux central et également comme antalgiques et antiinflammatoires.

La dose journalière souhaitable est d'environ 0,1 à 200 mg/kilo de poids corporel de préférence de 1 à 50 mg/kg, administrée en dose unique ou en doses divisées entre 2 et 4 fois par jour.

Une forme retard peut être également élaborée pour éviter les prises répétées.

Parmi les nombreux composés décrits dans l'invention, on notera les activités pharmacologiques particulièrement puissantes des dérivés selon la formule générale (I) dans laquelle R représente un radical alkyle linéaire substitués par un groupe $NR_1$ $R_2$ et plus spécialement des dérivés bis (amino-2 éthyloxyimino)-5,7... -bis (amino-3 propyloxyimino)-5,7... et bis (diéthylamino-2 éthyloxy-imino)-5,7 dibenzo [a,c] cycloheptène [5 H], substitués ou non en 6, ainsi que des mêmes dérivés oximes symétriques de la phényle-2 indane dione 1-3.

La présente demande concerne également un procédé de synthèse de dérivés de formule I.

Les composés répondant à la formule I s'obtiennent par réaction des β dicéto cycloalcènes correspondants soit le phényl-2 - (1H) indène dione - 1,3(2H) ou le dihydro-6,7 (5H)-dibenzo [a,c] cycloheptène dione-5,7 de formule (II) avec l'halogéno hydrate d'une hydroxylamine O-substituée de formule III selon le schéma réactionnel ci-dessous :

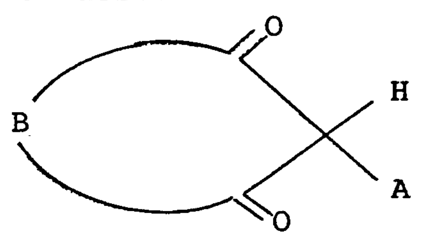

+ $H_2N$-OR, H Hal

(III)

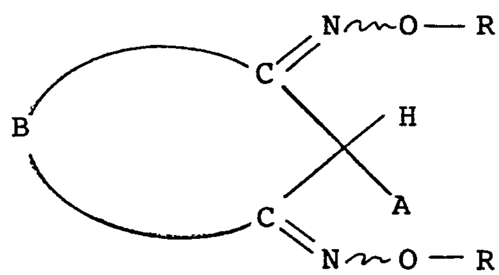

(I)

où R a les significations indiquées précédemment et Hal signifie le chlore ou le brome.

Cette réaction s'effectue dans un solvant qui peut être la pyridine ou un alcool comprenant de 1 à 3 atomes de carbone, anhydre ou aqueux ou encore un mélange de pyridine et d'alcool.

En milieu aqueux, il est souhaitable d'utiliser une base choisie parmi l'acétate de sodium ou de potassium, le carbonate de sodium ou de potassium, l'hydroxyde de calcium ou de magnésium.

De préférence, la réaction est conduite dans l'éthanol absolu à la température d'ébullition.

Quand elles ne sont pas commerciales, les hydroxylamines O-substituées peuvent être synthétisées par réaction du N-hydroxyphtalimide sur les dérivés halogénés Hal-R accessibles commercialement selon le schéma réactionnel ci-dessous :

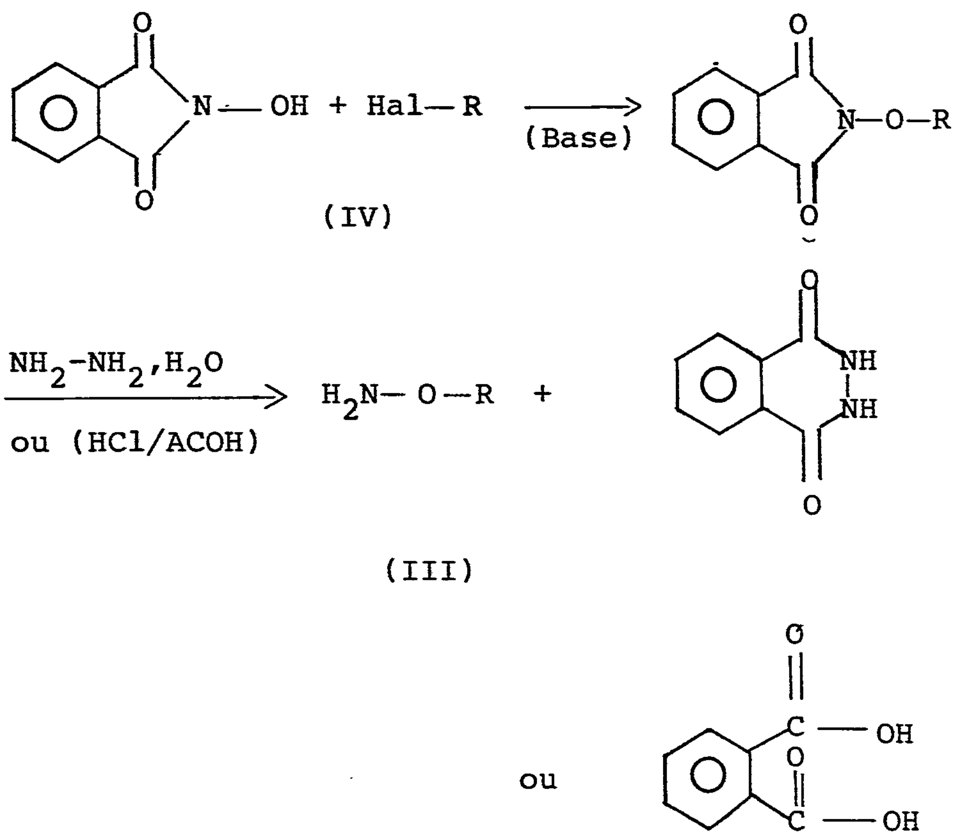


(IV)

(III)

ou

dans lequel on prépare le dérivé O substitué du N-hydroxyphtalimide V par réaction de ce dernier sur le dérivé Hal-R où Hal est un halogène de préférence le brome ou le chore en solution dans un solvant comme le diméthylformamide, l'acétonitrile, le diméthylsulfoxyde en présence d'une base comme la triéthylamine ou un carbonate alcalin à une température comprise entre 20° et 120°C.

De préférence, on opérera dans le diméthylformamide en présence de triéthylamine entre 100 et 110°C.

A partir du dérivé V, on prépare l'hydroxylamine O-substituée, par déblocage du groupement protecteur phtalimido. Ce déblocage peut être réalisé par hydrazinolyse en solution dans un alcool des premiers termes et à la température du reflux.

On utilise de préférence la quantité théorique d'hydrate d'hydrazine en solution dans l'éthanol. Par addition d'acide chlorhydrique concentré, on obtient le chlorhydrate de l'hydroxylamine O-substituée.

Les hydroxylamines O-substituées III, où R représente un groupe alkyl linéaire ou ramifié substitué par un groupement $-NR_1R_2$ peuvent être aussi avantageusement préparées selon un schéma réactionnel légérement différent.

L'alkylation du N-hydroxyphtalimide par un ω-ω' dibromoalkane, selon L. BAUER, J. Org. Chem. (1963), 28, p. 1604, permet d'accéder au produit de formule générale V, ω-bromé.

Ce dérivé, traité par une amine secondaire $HNR_1R_2$, conduit au produit de formule générale V, où R représente un groupe alkyl linéaire ou ramifié substitué par un groupement $-NR_1R_2$.

Cette réaction est conduite de préférence avec deux équivalents d'amine $HNR_1R_2$ ou avec un équivalent d'amine $HNR_1R_2$ et tout autre accepteur d'acide, choisi parmi la

pyridine, la triéthylamine, la collidine, le carbonate de sodium ou de potassium.

Cette réaction est menée en présence d'un solvant comme le benzène, le toluène, le xylène, l'acétonitrile, ou plus avantageusement l'acétone, la butanone-2, la méthyl-3 butanone-2, à une température comprise entre 40 et 130 °C, le plus souvent au reflux du solvant choisi.

Le déblocage du groupe protecteur phtalimido peut être réalisé par hydrazinolyse comme mentionné précédemment ou par hydrolyse acide, avec de l'acide chlorhydrique 6 N, seul ou en présence d'acide acétique, à la température du reflux.

L'hydroxylamine O-substituée de formule générale III ainsi obtenue est isolée sous forme de chlorhydrate.

Les composés de formule III peuvent être également préparés en faisant réagir le dérivé halogéné Hal-R (IV) avec un autre précurseur de l'hydroxylamine à azote protégé puis en éliminant le groupe protecteur de façon classique.

Parmi les autres précurseurs retenus, différents du N-hydroxyphtalimide, on peut citer l'acide benzohydroxamique, le N-hydroxyuréthane ou mieux le t. butyl N-hydroxy carbamate ou encore l'acétone oxime.

Lorsque on utilise un groupe isopropylidène ou α hydroxybenzylidène, la réaction s'effectue en présence d'un agent basique, par exemple un alcoolate de sodium dans l'alcool correspondant ou un carbonate alcalin dans un hydrocarbure aromatique de préférence le benzène.

Lorsque l'on utilise un groupe isopropylidène ou α hydroxy benzylidène ou encore carbalkoxy-, le groupement protecteur est éliminé par une hydrolyse acide.

Lorsque dans une formule III, R représente un groupement carboxyalkyl du type

$H_2N-O-(CH_2)_m-COOH$

il est nécessaire d'utiliser pour la réaction sur le N-hydroxyphtalimide un groupe protecteur de la fonction carboxylique plus précisément un ester d'alkyle inférieur choisi parmi les radicaux : méthyl, éthyl, propyl, isopropyl ou terbutyl ou un ester benzylique.

Après l'obtention du dérivé de formule (I) où R représente un motif

$-(CH_2)_m-COO$-alkyl inférieur

ou $-(CH_2)_m-COO$-benzyl

on peut obtenir le dérivé de formule (I) où R représente le motif

$-(CH_2)_m-COOH$

par hydrolyse alcaline des esters, méthyle, éthyle ou propyle. Cette hydrolyse peut être conduite dans un mélange hydroalcoolique contenant un léger excès d'un hydroxyde alcalin, la réaction étant conduite entre la température ordinaire et la température de reflux de préférence entre 40 et 60 degrés C. L'hydrolyse de l'ester tertiobutyl est réalisée de préférence par l'acide trifluoroacétique.

Dans le cas où R représente un motif

$(CH_2)_m-COO$ benzyl

l'élimination du groupe benzylique peut se faire par hydrolyse alcaline comme ci-dessus ou par hydrogénolyse en présence d'un catalyseur au Palladium en solution dans un alcanol inférieur de préférence l'isopropanol.

Lorsque dans la formule III

$NH_2-O-R$

R représente un radical hydrocarboné substitué par une amine primaire ou secondaire donc porteur d'un ou deux hydrogènes mobiles, il est nécessaire de protéger cette fonction, pendant la synthèse de l'hydroxylamine O substituée suivant le mode opératoire suivant :

si R = $Y-NH-R_8$

où Y est un motif hydrocarboné linéaire, ramifié ou cyclique et $R_8$ est l'hydrogéne ou un radical alkyl inférieur.

On fait réagir les quantités stoechiométriques d'un dérivé α halogéno ω amino ou alkylaminoalkyl et de chlorure de benzyloxycarbonyl.

La réaction est conduite dans un solvant organique ou dans l'eau en présence d'une base minérale et à température comprise entre 0 et 50 °C.

De préférence, on opérera en solution dans la soude normale contenant 10 % d'acétone et à température ordinaire.

Après évaporation du solvant, acidification et extraction, le dérivé N benzyloxycarbonylamino est mis en réaction selon le procédé précédemment décrit.

On aboutit alors aux dérivés de formule I dans lequel R comporte un groupe benzyloxycarbonyl protecteur de l'atome d'azote réactif.

On libère alors les fonctions amines par hydrogénolyse en solution dans un alkanol inférieur en présence d'un catalyseur au Palladium en présence d'hydrogéne entre 0 et 20 Bar de pression et à une température comprise entre 20° et l'ébullition, le milieu étant neutre ou légérement acide.

De préférence, on opérera en solution dans l'éthanol en présence d'un catalyseur au Palladium à 5 % sur charbon, en présence d'hydrogéne à pression atmosphérique avec la quantité théorique d'acide chlorhydrique pour réaliser le chlorhydrate des amines libérées.

Les exemples qui suivent permettent de décrire de façon non limitative l'étendue de la présente invention. Les produits obtenus ont été soumis à l'analyse élémentaire et correspondent aux pourcentages théoriques calculés à 0,3 % près.

La pureté des composés selon l'invention a été

contrôlée par chromatographie sur couche mince sur plaque Kieselgel F 254 avec les systèmes d'élution suivants :

A : Toluène 10 - Acide formique 1 - Formiate d'éthyle 10

B : n butanol 8 - Acide acétique 1 - Eau 1

C : Benzène 70 - Méthanol 0,5

D : n butanol 1 - acide acétique 1 - eau 1 - acétate d'éthyl 1

EXEMPLE 1 - BIS(ALLYLOXYIMINO)-5,7 DIHYDRO-6,7 (5H) DIBENZO [a,c] CYCLOHEPTENE

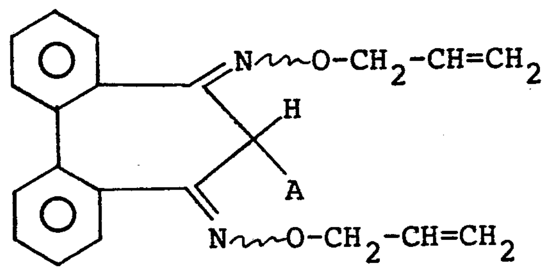

a) Allyloxyamine chlorhydrate

On ajoute 81,5 g (0,5 mole) de N hydroxyphtalimide à 300 ml de diméthylformamide puis introduit à température ordinaire 83 ml de triéthylamine. Sur la solution colorée obtenue, on coule 50 ml (0,6 mole) de bromure d'allyle puis porte à 100/110 °C pendant 5 heures, refroidit et filtre à température ordinaire le bromhydrate de triéthylamine. Le filtrat est évaporé à sec sous pression réduite, repris au chloroforme puis lavé à l'eau. Après évaporation, on obtient 95 % de O-allyloxyphtalimide - pF = 60 °C, que l'on dissout dans 450 ml d'éthanol dans lequel on coule en quelques minutes 27 ml d'hydrate d'hydrazine (0,55 mole).

On porte ensuite à reflux sous agitation vive pendant 3 heures, refroidit vers 0 °C puis coule lentement 6,4 ml d'acide chlorhydrique concentré. On laisse cristalliser à froid puis filtre le chlorhydrate d'allyloxyamine qu'on peut recristalliser dans l'alcool pour obtenir de beaux

cristaux blancs avec un rendement de 90 % par rapport à l'hydroxyphtalimide.

b) Bis(allyloxyimino)-5,7 dihydro-6,7 (5H)dibenzo [a,c] cycloheptène dione-5,7

A 250 ml d'éthanol contenant 12,67 g (57 mM) de dibenzo [a,c] dihydro-6,7 (5H) cycloheptène dione-5,7, on ajoute 12,5 g (0,114 mole) de l'allyloxyamine chlorhydrate préparé selon a et porte à reflux quelques heures jusqu'à disparition du produit de départ.

On évapore et purifie par chromatographie sur silice avec du benzène, on obtient ainsi le dérivé du titre constitué par le mélange des divers isomères Z,E ; E,E ; Z,Z. Le rendement est de 75 % sur la dione.

Les caractéristiques physico chimiques sont les suivantes : (RMN dans $CDCl_3$/TMS) : 7,4 ppm (s) 8 H (arom) 5,7/6,2 ppm, (m) 1H (<u>CH</u>-$CH_2$) 5,0/5,3 ppm (m) 2 H, (CH=<u>$CH_2$</u>) 4,6 ppm (m) 2 H, (-$OCH_2$) 4,25 ppm (s) 2 H, (-$CH_2$-) pour l'isomère symétrique.

L'isomère dissymétrique E-Z donne un massif complexe à 7,4 ppm, 8 H, (arom) et un singulet moins déblindé à 4,0 ppm, 2 H, (-$CH_2$). Le rapport des intensités des signaux du -$CH_2$- du cycle permet de déterminer le % respectif des isomères symétrique et dissymétrique.

IR (dans KBr) 1600 et 1645 $cm^{-1}$ fines, 1440 $cm^{-1}$ (F), 1030 et 930 $cm^{-1}$ (FF)=N-O-.

EXEMPLE 2 - BIS(METHOXYIMINO)-5,7-DIHYDRO-6,7 (5H) DIBENZO [a,c] CYCLOHEPTENE

A 6,5 g (29,3 mM) de dibenzo [a,c] dihydro-6,7 (5H) cycloheptène dione-5,7 dissous dans 160 ml d'éthanol, on ajoute 5 g (59,9 mM) de chlorhydrate de méthoxyamine commerciale. On traite ensuite dans les conditions de l'exemple 1-b. Le produit brut d'évaporation est recristallisé dans l'isopropanol. On obtient ainsi un premier jet de 57 % de produit blanc présentant un pF de 130°/136 °C contenant plus de 90 % d'un isomère pur (CCM

système A - Rf = 0,90 - la dicétone de départ est à Rf = 0,7). RMN : 7,4 ppm, (s), 8 H, (arom) - 4,20 ppm, (s), ($-CH_2-$)-isomère majoritaire 3,75 ppm, (s), traces du deuxième isomère - 3,85 ppm, (s), 6 H, ($-OCH_3$). Un deuxième jet de 20 % de pF = 140 °C constitué d'un isomère pur porte le rendement global à 77 %.

EXEMPLE 3 - BIS(ETHOXYIMINO)-5,7 DIHYDRO-6,7 (5H) DIBENZO [a,c] CYCLOHEPTENE

En suivant les conditions opératoires de l'exemple 1-b et en utilisant le chlorhydrate d'éthoxyamine, on obtient le dérivé du titre avec un rendement de 90 % présentant les deux taches des isomères symétrique et dissymétrique en CCM dans le système A, respectivement à Rf = 0,87 et 0,80 ou dans le benzène seul à Rf = 0,42 et 0,27. Le pF est de 112 °C.

Après plusieurs cristallisations dans l'alcool isopropylique, l'isomère de Rf le plus élevé se transforme progressivement en l'autre isomère de Rf le plus bas dont le pF est alors de 118 °C. RMN : 7,4 ppm, (s), 8 H, (arom)- 4,25 ppm, (s), 2 H ($-CH_2-$) - 4,20 ppm, (q), 4 H, ($O-\underline{CH_2}-CH_3$) - 1,25 ppm, (t), 6 H, ($O-CH_2-\underline{CH_3}$).

EXEMPLE 4 - BIS(n-PROPOXYIMINO)-5,7 DIHYDRO-6,7 (5H) DIBENZO [a,c] CYCLOHEPTENE

a) n propoxyamine chlorhydrate

En opérant selon l'exemple 1-a et en utilisant du bromo-1 propane à la place du bromure d'allyl, on obtient la n-propoxyamine, chlorhydrate sous forme de cristaux blancs de pF 155/6 °C avec un rendement de 80 % par rapport au phtalimide.

b) di(O-n-propyloxime) de la dihydro-6,7 (5H)dibenzo [a,c] cycloheptène dione-5,7

En opérant dans les conditions de l'exemple 1-b, on obtient après purification sur colonne de silice par le benzène, le dérivé du titre sous forme d'une huile jaune avec un rendement de 75 % par rapport au N- hydroxyphtalimide.

Le produit présente en CCM dans le système A une tache double à Rf 0,92.

Dans le benzène seul, la CCM permet de différencier deux taches d'importance identique à Rf 0,45 et 0,55. RMN : 7,4 ppm (s) et (m) au total 8 H (arom) - 4,25 et 4,05 ppm deux (s) d'intensité identique, 2 H au total ($-CH_2-$) - 4,0 ppm, (m), 4 H, ($O\underline{CH_2}-CH_2$) - 1,55 ppm (m) 4 H ($OCH_2-\underline{CH_2}-CH_3$), 0,9 ppm, (m), 6 H, ($OCH_2-CH_2-\underline{CH_3}$).

EXEMPLE 5 - BIS(CYANO-3 PROPOXYIMINO)-5,7 DIHYDRO-6,7 (5H)DIBENZO [a,c] CYCLOHEPTENE

a) Cyano-3 propoxyamine chlorhydrate

Selon les conditions opératoires de l'exemple 1-a et en utilisant le n-bromo-4 butyronitrile au lieu du bromure d'allyle, on obtient après cristallisation dans l'éther isopropylique le chlorhydrate de la O(cyano-3 propyl) hydroxylamine avec un rendement de 95 % par rapport à l'hydroxyphtalimide sous forme de cristaux blancs de pF = 134/136 °C.

b) Di[O-(cyano-3 propyl)oxime] de la dihydro-6,7 (5H) dibenzo [a,c] cycloheptène dione-5,7

En utilisant les conditions opératoires de l'exemple 1-b avec l'hydroxylamine obtenue précédemment en 5-a, on obtient le dérivé du titre que l'on purifie par chromatographie sur colonne de silice par un mélange chloroforme-acétone. On obtient ainsi le dérivé du titre sous forme d'une huile épaisse avec un rendement de 70 % (à partir de la dione) présentant en CCM une tache légérement dédoublée dans le système A à Rf = 0,65 et deux spots d'intensité identique à Rf 0,05 et 0,15 en CCM dans le système benzène 100/méthanol 0,5 correspondant aux isomères symétrique et dissymétrique.

EXEMPLE 6 - BIS(TERBUTOXYIMINO)-5,7-DIHYDRO-6,7 (5H) DIBENZO [a,c] CYCLOHEPTENE

En suivant les conditions de l'exemple 1-b à partir

du chlorhydrate de t.butoxyamine,on obtient 82% du dérivé du titre, sous forme de cristaux blancs de pF 181-3 °C présentant une seule tache en CCM dans le système A de Rf = 0,95 et également un seul spot par CCM dans le benzène seul de Rf = 0,85. RMN : 7,4 ppm (s), 8 H (arom) - 4,2 ppm,(s), 2 H($-CH_2-$) - 1,25 ppm,(s), 18 H, (t. but.)

EXEMPLE 7 - BIS(CYCLOHEXYLMETHYLOXYIMINO)-5,7-DIHYDRO-6,7 (5H)DIBENZO [a,c] CYCLOHEPTENE

a) Cyclohexylméthyloxyamine chlorhydrate

En opérant selon l'exemple 1-a et en utilisant le bromométhylcyclohexane à la place du bromure d'allyle, on obtient avec un rendement de 70 % par rapport au phtalimide le chlorhydrate de O-cyclohexyméthyl hydroxylamine sous forme de cristaux blancs de pF = 173 °C.

b) Di(O-cyclohexylméthyloxime) de la dihydro-6,7 (5H)dibenzo [a,c] cycloheptène dione-5,7

En opérant dans les conditions de l'exemple 1-b, on obtient le dérivé du titre avec un rendement de 91 % sur la dione. Une recristallisation dans le chloroforme aboutit à un premier jet d'un isomère pur de pF 97/99 °C. RMN : 7,4 ppm, (s), 8 H, (arom) - 4,2 ppm, (s), 2 H, ($-CH_2-$) - 3,90 ppm, (d), (J = 6Hz), 4 H, ($-O\underline{CH_2}-$) - 1,8 à 1,0 ppm, (m), 22 H.

EXEMPLE 8 - BIS(BENZYLOXYIMINO)-5,7 DIHYDRO-6,7 (5H) DIBENZO [a,c] CYCLOHEPTENE

En suivant le mode opératoire décrit à l'exemple 1-b et en utilisant le chlorhydrate de O-benzylhydroxylamine à la place du chlorhydrate d'allyloxyamine, on obtient le dérivé du titre avec un rendement de 81 % par rapport à la dione de départ. pF = 112/114 °C présentant en CCM un spot de Rf 0,95 dans le système A et de 0,80 dans un mélange benzène/méthanol 70/0,2.

EXEMPLE 9 - BIS(PHENYL-2 ETHOXYIMINO)-5,7 DIHYDRO-6,7 (5H)DIBENZO [a,c] CYCLOHEPTENE

a) O-phénéthyl hydroxylamine chlorhydrate

En opérant comme indiqué à l'exemple 1-a avec du bromure de phénéthyle au lieu du bromure d'allyle, on obtient le chlorhydrate de (phényl)-2 éthoxyamine avec un rendement de 91 % sous forme de cristaux blancs.

b) Bis(phényl-2 éthoxyimino)-5,7 dihydro-6,7 (5H)dibenzo [a,c] cycloheptène

En utilisant la O-phénéthyl hydroxylamine préparée selon a, dans les conditions de l'exemple 1-b, on obtient après chromatographie sur silice par le benzène avec un rendement de 85 % le dérivé du titre à l'état pur sous forme d'une huile épaisse, mélange des isomères : RMN : 7,4 ppm, (s), 8 H, (arom) - 7,2 ppm, (s), 10 H, (arom) - 4,2 et 3,9 ppm, (2 s), ($-CH_2-$) - 4,25 ppm, (m), 4 H, ($OCH_2$) - 2,9 ppm, (m), 4 H, ($O-CH_2-\underline{CH_2}-$).

EXEMPLE 10 - BIS(ETHOXYCARBONYLMETHYLOXYIMINO)-5,7 DIHYDRO-6,7 (5H)DIBENZO [a,c] CYCLOHEPTENE

a) O-éthoxycarbonylméthyl hydroxylamine chlorhydrate

En utilisant du bromoacétate d'éthyle à la place du bromure d'allyle et en suivant les conditions de l'exemple 1-a, on obtient le chlorhydrate d'éthoxycarbonylméthyloxyamine avec un rendement de 91 % sous forme de cristaux blancs de pF = 106 °C.

b) Di(O-éthoxycarbonylméthyloxime) de la dihydro-6,7 (5H)dibenzo [a,c] cycloheptène dione-5,7

En opérant dans les conditions de l'exemple 1-b à partir de l'hydroxylamine précédente, on obtient après purification chromatographique dans le chloroforme, 55 % du dérivé du titre sous forme de cristaux blancs de pF 95 °C présentant en CCM dans le système chloroforme/acétone 70/1 deux taches de Rf 0,60 et 0,65 correspondant aux deux isomères. RMN : 7,4 ppm, (s), 8 H, (arom) - 4,65 ppm, (s), 4 H ($-O\underline{CH_2}COO$) - 4,45 ppm (d) et 4,0 ppm (s), 2 H, ($-CH_2-$) mélange des isomères - 4,1 ppm, (q), 4 H, ($-\underline{CH_2}CH_3$) - 1,15 ppm, (t), 6 H, ($OCH_2$ $\underline{CH_3}$).

EXEMPLE 11 - BIS(TERBUTOXYCARBONYLMETHOXYIMINO)-5,7

DIHYDRO-6,7 (5H)DIBENZO [a,c] CYCLOHEPTENE

a) O-t-butoxycarbonylméthyl hydroxylamine chlorhydrate

En utilisant le bromo acétate de t. butyle dans les conditions de l'exemple 1-a, on obtient le chlorhydrate de ter-butoxycarbonylméthyloxyamine sous forme de cristaux blancs de pF 98 °C avec un rendement quantitatif.

b) bis(terbutoxycarbonylméthoxyimino)-5,7 dihydro-6,7 (5H)dibenzo [a,c] cycloheptène

On opère dans les conditions de l'exemple 1-b à partir de l'hydroxylamine préparée précédemment et l'on obtient le dérivé du titre avec un rendement de 65 % à partir de la dione sous forme de cristaux blancs de pF 105-107 °C ; RMN : 7,4 ppm, (m), 8 H, (arom) - 4,5 ppm, (s), 4 H, (O$\underline{CH_2}$CO) - 4,3 ppm (d) et 4,1 ppm (s), (2 H), ($-CH_2-$) mélange des isomères - 1,40 ppm (s) 18 H (t but). Le produit présente une seule tache en CCM dans le système A de Rf = 0,87.

EXEMPLE 12 - BIS(ETHOXYCARBONYLPROPYLOXYIMINO)-5,7 DIHYDRO-6,7 (5H)DIBENZO [a,c] CYCLOHEPTENE

a) O(éthoxycarbonylpropyl) hydroxylamine chlorhydrate

En opérant dans les conditions de l'exemple 1-a à partir du bromo-4 butyrate d'éthyle, on obtient le chlorhydrate de l'éthoxycarbonylpropyloxyamine sous forme de cristaux blancs de pF 99 °C avec un rendement de 75 % à partir du N-hydroxyphtalimide.

b) bis(éthoxycarbonylpropyloxy)-5,7 dihydro-6,7 (5H) dibenzo [a,c] cycloheptène

En opérant selon 1-b avec l'hydroxylamine précédemment préparée, on obtient le dérivé du titre avec un rendement de 80 % sous forme d'une huile épaisse, présentant un seul spot en CCM dans le système A et les deux taches des isomères symétrique et dissymétrique en CCM dans le benzène respectivement à Rf 0,55 et 0,60. RMN : 8,4 ppm (s large), 8 H, (arom) - 4,25 à 3,90 ppm (m complexe), 10 H, ($-\underline{CH_2}-$, O$\underline{CH_2}$-$CH_2$,-COO$\underline{CH_2}$) - 2,5 à

1,8 ppm, (m), 8 H, ($\underline{CH_2}$-$\underline{CH_2}$-CO) - 1,25 ppm, (t), 6 H, (O-$CH_2$-$\underline{CH_3}$).

EXEMPLE 13 - BIS(HYDROXYCARBONYLMETHYLOXYIMINO)-5,7 DIHYDRO-6,7 (5H)DIBENZO [a,c] CYCLOHEPTENE

On ajoute 13 mM (6,6 g) d'ester ter-butylique, décrit à l'exemple 11, à 40 ml d'acide trifluoroacétique. Après deux heures à température ordinaire, on évapore sous pression réduite et reprend à l'éther puis à l'éther de pétrole.

On obtient le dérivé du titre avec un rendement quantitatif sous forme de cristaux blancs de pF = 185 °C présentant en CCM un seul spot de Rf 0,45 dans le système A et un spot légérement dédoublé dans le système B de Rf 0,70 ; RMN : 7,4 ppm, (s), 8 H, (arom) - 4,70 ppm, (s), 4 H, (-O-$CH_2$-CO) - 4,5 et 4,1 ppm (d) et (s), 2 H, (-$CH_2$-) isomères à 10,8 ppm 2 H échangeables.

EXEMPLE 14 - BIS(PYRIDINYL-3 METHYLOXYIMINO)-5,7 DIHYDRO-6,7 (5H)DIBENZO [a,c] CYCLOHEPTENE DICHLORHYDRATE

a) O-(pyridinyl-3 méthyl) hydroxylamine chlorhydrate

On opère selon les conditions de l'exemple 1-a en utilisant le chlorhydrate de chlorométhyl-3 pyridine. En fin d'opération toutefois et avant la reprise au chloroforme, on alcalinise la solution aqueuse à la soude pour obtenir le N[picolyloxy-3] phtalimide, base.

Le traitement habituel aboutit au chlorhydrate de O(picolyl-3) hydroxylamine, cristaux blancs de pF 182 °C.

b) bis(pyridinyl-3 méthyloxyimino)-5,7 dihydro-6,7(5H) dibenzo [a,c] cycloheptène dichlorhydrate

En opérant suivant les conditions de l'exemple 1-b et en utilisant l'hydroxylamine précédemment préparée, on obtient le bis[pyridinyl-3 méthyloxyimino]-5,7 dihydro-6,7 (5H)dibenzo [a,c] cycloheptène, qu'on purifie par chromatographie sur alumine dans le chloroforme.

Le produit pur est dissous dans 2 volumes d'éthanol

et additionné de la quantité théorique d'acide chlorhydrique anhydre, par addition d'éther, on obtient le dérivé du titre avec un rendement de 70 % par rapport à la dione sous forme de cristaux blancs de pF 130 °C présentant en CCM dans le système B un seul spot de Rf = 0,60 (la dione est à 0,95) et également une seule tache par CCM sur alumine dans le chloroforme.

RMN : 9 à 7,4 ppm, (m), 18 H, (arom) - 5,4 ppm, 4 H, (s), $(-OCH_2-)$ - 5,2 et 4,4 ppm, (s), 2 H, $(-CH_2-)$.

EXEMPLE 15 - BIS(DIETHYLAMINO-2 ETHYLOXYIMINO)-5,7 DIHYDRO-6,7 (5H)DIBENZO [a,c] CYCLOHEPTENE DICHLORHYDRATE

a) Chlorhydrate de la diéthylamino-2-éthoxyamine à partir de l'isopropylidéne hydroxylamine

On dissout 73 g d'acétoneoxime dans 250 ml de benzène, ajoute 17,5 g de chlorhydrate de diéthylamino-chloréthane puis introduit avec précaution et sous agitation vive à température ordinaire 350 g de carbonate de potassium sec puis porte à reflux pendant 10 heures.

La suspension est filtrée et la solution organique est extraite par un excès d'acide chlorhydrique dilué. La solution chlorhydrique est ensuite portée à 60 °C sous bonne agitation et la pression est baissée progressivement afin de distiller l'acétone fournie et l'excès d'eau.

Par refroidissement et ajout d'éthanol, on fait cristalliser le dichlorhydrate de diéthylaminoéthoxyamine.

b) En utilisant les conditions de l'exemple 14-b avec le chlorhydrate de diéthylamino-2 éthyloxyamine, on obtient le dérivé du titre sous forme d'une huile jaune.

Par dissolution dans l'éthanol et ajout de la théorie d'acide chlorhydrique anhydre et reprise à l'éther,on obtient le dichlorhydrate de bis(diéthylamino-2 éthyloxyimino)-5,7 dihydro-6,7 (5H)dibenzo [a,c] cycloheptène sous forme de cristaux blancs de pF = 173 °C présentant

en CCM dans le système B deux taches de Rf 0,38 et 0,25 correspondant aux deux isomères. RMN : 7,5 ppm, (m), 8 H, (arom) - 4,5 ppm, (m), 4 H, ($O-\underline{CH_2}-CH_2$) - 4,4 et 3,9 ppm, (s), 2 H, ($-CH_2-$) isomères - 3,5 à 2,8 ppm, (m), 12 H, ($-N-CH_2-$) - 1,2 ppm, (t), 6 H, ($-CH_2-\underline{CH_3}$).

On peut préparer les sels d'acide organique de la base préparée selon l'exemple 15 en mélangeant dans un rapport molaire de 1 à 2 la base et les acides organiques usuellement utilisés pour la salification des molécules à activité thérapeutique à caractère basique, on obtient ainsi les dérivés suivants :

<u>EXEMPLE 16 - BIS (DIETHYLAMINO-2 ETHYLOXYIMINO) -5,7 DIHYDRO - 6,7 (5H) DIBENZO [a,c] CYCLOHEPTENE DIMALEATE</u>

Par salification dans l'isopropanol, on obtient le dérivé du titre sous forme d'une masse amorphe de pF = 74°C.

<u>EXEMPLE 17 - BIS (DIETHYLAMINO-2 ETHYLOXYIMINO) -5,7 DIHYDRO - 6,7 (5H) DIBENZO [a,c] CYCLOHEPTENE DITARTRATE</u>, que l'on recristallise dans l'isopropanol contenant 1 % d'eau - beaux cristaux blancs de p F = 60°C.

On obtient de la même façon le difumarate (pF = 70°C).

<u>EXEMPLE 18 - BIS(AMINO-2 ETHYLOXYIMINO)-5,7 DIHYDRO-6,7 (5H)DIBENZO [a,c] CYCLOHEPTENE DICHLORHYDRATE</u>

a) <u>Chlorhydrate de la benzyloxycarbonylamino-2 éthyloxyamine</u>

A 0,45 mole (92,2 g) de bromo-2 éthylamine en solution dans 1 litre de soude normale, on ajoute lentement 0,46 mole (78,5 g) de chlorure de benzyloxycarbonyl en solution dans 1 volume d'acétone. En fin de réaction (environ 48 heures), on évapore l'acétone sous pression réduite puis acidifie entre pH 1 et 2 et extrait à l'éther. Après évaporation, on obtient le benzyloxycarbonylamino bromo-2 éthane. Ce produit est mis en réaction avec le N-hydroxyphtalimide selon les modalités de l'exemple 1-a, on obtient ainsi le N-benzyloxycarbonylamino-2 éthyloxyphtalimide avec un rendement de 73 % sous forme de cristaux blancs de pF = 95 °C. Après traitement à l'hydrazine selon les conditions de l'exemple 1-a, on obtient le chlorhydrate de la benzyloxycarbonylamino-2 éthyloxyamine de pF 178 °C (Rendement/phtalimide = 95 %).

b) Bis(benzyloxycarbonylamino-2 éthyloxyimino)-5,7 dihydro-6,7 (5H)dibenzo [a,c] cycloheptène

En suivant les conditions opératoires de l'exemple 1-b et en utilisant le chlorhydrate de l'oxyamine préparée ci-dessus, on obtient le dérivé du titre avec un rendement de 75 % après une purification sur colonne de silice dans le chloroforme. Le produit, mélange des isomères, se présente sous forme d'une huile épaisse :
RMN : 7,4 ppm, (m), 18 H, (arom) - 5,1 ppm, (s), 4 H, ($COO\underline{CH_2}$) - 4,8 et 4,0 ppm, (m), 2 H, ($-CH_2-$) - 4,2 ppm, (t), 4 H, ($-O\underline{CH_2}-CH_2$) - 3,5 ppm, 4 H, (q), ($-NCH_2-$).

c) dichlorhydrate du bis(amino-2 éthyloxyimino)-5,7 dihydro-6,7 (5H)dibenzo [a,c] cycloheptène

On soumet le produit obtenu au paragraphe précédent à une hydrogénolyse dans l'éthanol chlorhydrique en présence de Palladium à 5 % sur charbon. La solution alcoolique diluée à l'éther laisse cristalliser le dérivé du titre sous forme de beaux cristaux blancs de pF 210 °C présentant en CCM dans le système B une tache de Rf 0,50
RMN : 7,4 ppm, (s), 8 H, (arom) - 4,5 ppm, (s), et 4,1 ppm, (m), 2 H, $-CH_2-$ (isomères) - 4,3 ppm, (m), 4 H, ($-O\underline{CH_2}-CH_2$) - 3,2 ppm, (m), 4 H, ($-NH_2-\underline{CH_2}$).

EXEMPLE 19 - BIS(AMINO-3 PROPYLOXYIMINO)-5,7 DIHYDRO-6,7 (5H)DIBENZO [a,c] CYCLOHEPTENE DICHLORHYDRATE

a) Chlorhydrate de la benzyloxycarbonylamino-3 propyloxyamine

On opère dans les conditions de l'exemple 18-a en utilisant la bromo-3 propylamine à la place de la bromo-2 éthylamine. On obtient ainsi le bromo-3 benzyloxycarbonylamino propane. Mis en réaction avec le N-hydroxyphtalimide dans les conditions de l'exemple 1-a, le dérivé précédent aboutit au N-benzyloxycarbonylamino-3 propyloxy phtalimide.

Après traitement habituel à l'hydrazine, on, obtient le chlorhydrate de la benzyloxycarbonylamino-3 propyloxyamine, sous forme de cristaux blancs de pF = 180°C avec un rendement de 85 %.

b) Bis(benzyloxycarbonylamino-3 propyloxyimino)-5,7 dihydro-6,7 (5H)dibenzo [a,c] cycloheptène

A partir de l'oxyamine préparée précédemment et dans les conditions de l'exemple 1-b, on obtient le dérivé du titre sous forme d'un solide amorphe. RMN : 7,4 ppm, (2s), 18 H, (arom) - 5,0 ppm, (s), 4 H, ($-CH_2-OCO$) - 4,2 à 4,05 ppm, (t), 6 H, ($-CH_2-$) isomères et ($N-O-\underline{CH_2}$) - 3,2 ppm, (q), 4 H, ($-\underline{CH_2}-NH-$) - 1,8 ppm, (q), 4 H, ($-CH_2-\underline{CH_2}-CH_2-$).

c) Dichlorhydrate du bis(amino-3 propyloxyimino)-5,7 dihydro-6,7 (5H)dibenzo [a,c] cycloheptène

En opérant selon l'exemple 18-c, avec le produit obtenu au paragraphe précédent, on obtient le dérivé du titre avec un rendement de 85 % sous forme de cristaux blancs de pF = 208°C présentant en CCM dans le système B une seule tache de Rf 0,50 et de Rf = 0 dans le système A. RMN : 8 à 8,5 ppm, (m), H échangeables - 7,4 ppm, (s), large, 8 H, (arom) - 4,2 ppm, (m), 6 H, ($-CH_2-$) ($-O\underline{CH_2}-CH_2-$) - 2,9 ppm, (m), 4 H, ($-\underline{CH_2}-NH_2-$) - 2,0 ppm, (m), 4H ($-CH_2-\underline{CH_2}-CH_2$).

EXEMPLE 20 - BIS(PIPERIDINYL-1 ETHOXYMINO)-5,7 DIHYDRO-6,7 (5H)DIBENZO [a,c] CYCLOHEPTENE DICHLORHYDRATE

a) N(pipéridinyl-1 éthoxy) phtalimide :

On ajoute 21,3 g (0,25 mole) de pipéridine à une solution de 54 g (0,2 mole) de N(bromo-2-éthoxy) phtalimide, dans 800 ml de méthyléthylcétone, en présence de 31,5 g (0,3 mole) de carbonate de sodium

et 1g d'iodure de sodium.

On porte à reflux pendant 3 heures, sous agitation vive, puis réfrigère pour filtrer et évaporer le filtrat. Le résidu est repris au chloroforme pour des lavages aqueux. On obtient avec un rendement de 70 %, le dérivé du titre, présentant en CCM une tache de Rf = 0,69 dans le système B. Le pF est de 65-9°C.

b) O-(pipéridinyl-1 éthyl) Hydroxylamine dichlorhydrate

On ajoute 35 ml d'acide chlorhydrique à 35 % à une solution de 27,4 g (0,1 mole) du dérivé préparé précédemment dans 60 ml d'acide acétique. On porte à reflux pendant 30 minutes, puis réfrigère pour filtrer et évaporer le filtrat.

Le résidu est repris plusieurs fois à l'éthanol, puis empâté dans l'éther isopropylique. Après filtration et séchage, on obtient avec un rendement de 75 %, le dérivé du titre, présentant en CCM une tache de Rf = 0,36 dans le système B. Le pF est de 178-84°C.

c) dichlorhydrate du bis(pipéridinyl-1 éthoxyimino)-5,7 dihydro-6,7 (5H)dibenzo [a,c] cycloheptène

En suivant les conditions opératoires de l'exemple 1-b et en utilisant l'hydroxylamine préparée précédemment, on obtient le bis(pipéridinyl-1 éthoxyimino)-5,7 dihydro-6,7 (5H)dibenzo [a,c] cycloheptène, après une chromatographie sur alumine dans le chloroforme.

Le produit pur est dissous dans 2 volumes d'éthanol et additionné de la quantité théorique d'acide chlorhydrique en solution éthérée. Par addition d'éther, on obtient le dérivé du titre avec un rendement de 55 % par rapport à la dione, sous forme de cristaux de pF = 115-20°C, présentant en CCM dans le système B une seule tache de Rf = 0,55.

RMN : 7,4 ppm, (s) large, 8 H, (arom) - 4,2 ppm, (m), 6 H, $(-CH_2-)$ $(= OCH_2-)$ - 3,25 à 4 ppm, (m), 12 H, $(-CH_2N-)$ - 1,6 ppm, (s) large, 12 H, ($CH_2$, pipéridine).

EXEMPLE 21 - BIS(DIETHYLAMINO-2 ETHYLOXYIMINO)-1,3 PHENYL-2-(1H)INDENE [2H] - DICHLORHYDRATE

On ajoute 7,12 g (32 mM) de phényl-2 [1H] indène - (2H) dione - 1,3 (ou phénylindanedione) à 200 ml de pyridine puis introduit 13,8 g (67 mM) de chlorhydrate de diéthylaminoéthoxyamine préparé au paragraphe a) de l'exemple 15 puis chauffé à 60/65°C pendant 15 heures sous agitation, on maintient encore l'agitation pendant 48 heures à température ambiante.

On filtre le précipité, le lave soigneusement à l'éther, on obtient le dérivé du titre sous forme de cristaux blancs de pF = 187/189°C présentant un seul spot de Rf = 0,40 en CCM sur silice dans le système B et également un seul spot dans le système : chlorure de méthylène 10/acétonitrile 20/diéthylamine 0,5 - de Rf = 0,20. RMN (dans $D_2O$) : 7,9 et 7,7 ppm (m) couplé, 4H, arom de l'indène ; 7,30 ppm 5H (s) large phényl-2 ; 5,3 ppm (s) 1H en 2. 4,45 ppm (m), 4H, 2 x N-O-$\underline{CH_2}$ - ; 3,5 ppm (m) 4H, 2 x -N-$\underline{CH_2}$ ; 3,0 ppm (q) 8H, 4 x -N-$\underline{CH_2}$ - $CH_3$ ; 1,20 ppm (t) 12H, 4 x -N-$CH_2$-$\underline{CH_3}$.

EXEMPLE 22 - BIS (DIETHYLAMINO-2 ETHYLOXYIMINO) - 1,3 (1H) INDENE (1H) - DICHLORHYDRATE

On ajoute 4,25 g (29 mM) de (1H) - indène - [2H] dione - 1,3 à 150 mL d'éthanol puis introduit 12,1 g (59 mM) de chlorhydrate de diéthylaminoéthoxyamine (exemple 15, a)) dissout dans 50 ml d'éthanol. On maintient à 60°C pendant 8 heures sous agitation puis continue l'agitation à 45°C pendant encore 20 heures. On filtre le produit cristallisé, lave à l'éther et recristallise dans 50 volumes d'éthanol.

On obtient ainsi le dérivé du titre avec un rendement de 75 % sous forme de cristaux blancs de pF = 255°C présentant un seul spot de Rf = 0,20 en CCM sur silice dans le système D.

Son spectre de RMN enregistré sur la base en solution

chloroformique deuterée donne les signaux suivants : 8 à 7,5 ppm (m) 4H arom ; 4,5 ppm (t) 4H, 2 x N-O$\underline{CH_2}$- ; 4,2 ppm (s) 2H en 2 ; 3,5 ppm (m) 4H, 2 x -N-$CH_2$ ; 3,0 ppm (q) 8$\underline{H}$, 4 x -N$\underline{CH_2}$-$CH_3$ ; 1,20 ppm (t) 12H, 4 x N-$CH_2$-$\underline{CH_2}$.

EXEMPLE 23 - BIS (MORPHOLINO-2 ETHYLOXYIMINO) - 1,3 (1H INDENE (2H), DICHLORHYDRATE

On opère dans les conditions de l'exemple 22 en employant 2 équivalents de chlorhydrate de morpholino-2 éthyloxyamine (synthétisé selon BRIT. PAT. 842968).

Après deux cristallisations dans l'éthanol on obtient le dérivé du titre avec un rendement de 60% sous forme de cristaux blancs de pF = 254°C, présentant un seul spot de Rf = 0,30 en CCM dans le système B et de Rf = 0,30 dans le système D. En RMN on obtient les signaux suivants : De 7,7 à 7,4 ppm (m) 4H arom ; 4,9 ppm 1H ; 4,4 ppm (t) 4H -NO$CH_2$ ; 3,7 ppm (8H) 2 x $CH_2OCH_2$ ; 2,75 ppm -N-$CH_2$.

EXEMPLE 24 - BIS (MORPHOLINO- 2 ETHYLOXYIMINO) -1,3 PHENYL-2 (1H) INDENE (2H), DICHLORHYDRATE

On opère dans les conditions de l'exemple précédent en employant la phényl-2 indanedione au lieu d'indanedione.

On peut également opérer dans la pyridine au lieu d'éthanol. Le produit brut filtré en fin d'opération est traité par un excès d'éther chlorhydrique pour transformer la totalité du monochlorhydrate brut en dichlorhydrate.

On obtient ainsi le dérivé du titre, avec un rendement de 40 % sous forme de cristaux blancs de pF = 202°C, présentant en CCM un seul Rf = 0,50 dans le système D et de 0,35 dans le système B. Le spectre de RMN enregistré dans $D_2O$ présente les signaux caractéristiques ci-après : De 7,7 à 7,4 ppm 2 (m) 4H indène ; 7 ppm (s) large 5H - phényl ; 4,89 (s) 1H en position 2 ; 4,3 (m) 4H, 2 x N-O-$\underline{CH_2}$ - ; de 3,8 à 3,2 ppm 2 (m) non résolus,

et 2,60 ppm (m) non résolu.

EXEMPLE 25 - BIS [PIPERIDIN-1YL-2 ETHYLOXYIMINO] -1,3 PHENYL-2 (1H) INDENE (2H), DICHLORHYDRATE

On dissout 11,55 g (52 mM) de phényl-2 indanedione-1-3 dans 330 mL de pyridine, puis ajoute 23,9 g (110mM) de dichlorhydrate de piperidin-1yl-2 éthyloxyamine et chauffe 14 heures à 70°C.

En traitant de la façon habituelle on obtient le dérivé du titre avec un rendement de 65 % sous forme de cristaux blancs de pF = 219°C. Le produit présente en CCM sur silice un seul spot de Rf = 0,40 dans le système D. RMN dans $D_2O$ - 7,7 à 7,4 ppm 2 (m) 4H arom ; 7,0 (m) 5H phényl ; 4,9 ppm (s) 1H en position - 2 ; 4,2 ppm (m) 4H, 2 x N-O-$\underline{CH_2}$ ; 3,1 à 2,8 ppm (m) 8H, 4 x $-N-CH_2$ ; 2,2 ppm (t) 4H, 2 x N-$\underline{CH_2}$ - $CH_2$ - O ; 1,4 à 1,15 ppm (m) 12H 6 x - $CH_2$-

EXEMPLE 26 - BIS [PIPERIDIN-1YL-2 ETHYLOXYIMINO] - 1,3 (METHOXY-4) PHENYL-2 (1H) INDENE (2H), DICHLORHYDRATE

On utilise les conditions de l'essai précédent en utilisant de la (méthoxy-4) phényl-2 indanedione - 1,3 préparée selon J. Méd. Chem. (1985) - 28 (11) 1591 au lieu de phenyl-2 indanedione - 1,3.

Le produit brut est repris dans un excès d'éther chlorhydrique et l'on obtient le dérivé du titre avec un rendement de 45 % sous forme de cristaux blancs de pF = 180/185°C (décomposition), présentant un seul spot en CCM sur silice de Rf = 0,35 dans le système B et de Rf = 0,55 dans le système D. Le spectre de RMN est comparable à celui de l'exemple 25 avec un singulet supplémentaire à 3,8 ppm = 3H - $OCH_3$.

EXEMPLE 27 - BIS (DIETHYLAMINO-2 ETHYLOXYIMINO) - 1,3 (CHLORO-4) PHENYL-2 (1H) INDENE (2H) DICHLORHYDRATE

On dissout 30,75 g (0,15 M) de chlorhydrate de diéthylamino éthoxyamine dans 450 ml de pyridine anhydre puis introduit 19,25 g (75 mM) de (chloro-4) phényl-2

indanedione (préparé à partir du phtalide et de chlorobenzaldéhyde selon J. of Organ. Chem. 26, 3580 (1961). On agite à 100°C pendant 4 heures, puis une nuit à température ambiante. On évapore la pyridine sous pression réduite et reprend par de l'acide chlorhydrique dilué, lave à l'éther , puis reprécipite la base en alcalinisant par la soude. On l'extrait à l'éther, lave à l'eau et évapore. L'huile résiduelle est chromatographiée sur silice dans le chloroforme.

La base purifiée obtenue avec un rendement de 78 % est convertie en chlorhydrate par traitement dans l'éthanol avec de l'éther chlorhydrique. On obtient le dérivé du titre sous forme de cristaux de pF = 204°C présentant un seul spot en CCM sur silice de Rf = 0,40 dans le système B. Son spectre de RMN enregistré sur la base en solution dans $CDCl_3$ donne des signaux caractéristiques comparables à ceux enregistrés dans l'exemple 21 avec un singulet à 7,15 ppm au lieu de 7,30, 4H, -parachlorophényl.

EXEMPLE 28 - BIS (DIETHYLAMINO-2 ETHYLOXYIMINO) -1,3 (METHYL-4) PHENYL-2 (1H) INDENE (2H) DICHLORHYDRATE

On dissout 18,9 g (80 mM) de (méthyl 4)-phényl-2 indanedione (préparée à partir du phtalide et de tolualdéhyde selon J. of Organic Chem. 26, 3580 (1961) dans 450 mL de pyridine, ajoute 32,8 g (160 mM) de chlorhydrate de diéthylaminoéthoxyamine et porte à reflux pendant 4 heures.

On traite ensuite selon le protocole décrit à l'exemple 27. La base purifiée obtenue avec un rendement presque quantitatif est convertie en dichlorhydrate dans l'éther. On obtient le dérivé du titre sous forme de cristaux blancs de pF = 188°C présentant en CCM sur silice un seul spot de Rf = 0,35 dans le système B. Le spectre de RMN enregistré sur la base dans $CDCl_3$ donne les déplacements chimiques suivants par rapport au TMS :

7,75 et 7,50 ppm 2 (m) 4H indène ; 7,0 ppm (s) 4H phényl ; 5,15 ppm (s) 1H, CH en 2 ; 4,5 ppm (m) 4H 4 x = N-O-<u>$CH_2$</u> - ; 3,25 et 2,75 ppm 2 (m) 12H 6 x (N-$CH_2$) ; 2,30 ppm (s) 3H - $CH_3$-phenyl ; 1,20 ppm (9) 12H, 4 x - $CH_2$<u>$CH_3$</u>.

<u>EXEMPLE 29 - BIS (DIETHYLAMINO-2 ETHYLOXYIMINO) - 1,3 (METHYLENEDIOXY -3,4) PHENYL-2 (1H) INDENE (2H) DI-CHLORHYDRATE</u>

On opère de la façon précédente (exemple 28) en utilisant 18,6 g (70 mM) de (méthylènedioxy - 3,4) phényl-2 indanedione - 1,3 et 28,7 g (0,14 M) de chlorhydrate de diéthylaminoéthyloxyamine ; toutefois, la purification de la base est effectuée par chromatographie sur alumine par le chloroforme. Après conversion en chlorhydrate, on obtient le dérivé du titre avec un rendement de 85 % sous forme de cristaux de pF = 166°C et de Rf = 0,30 par CCM dans le système B.

<u>EXEMPLE 30 - BIS (ETHOXYCARBONYLMETHYLOXYIMINO) -1,3 PHENYL-2 (1H) INDENE (2H)</u>

On ajoute 12 g (100 mM) d'éthoxycarbonylméthyloxyamine chlorhydrate (voir exemple 10 a))à 50 mMoles de phényl-2 (1H) indène (2H) dione - 1,3 dans 200 ml d'éthanol et porte à reflux jusqu'à disparition du produit de départ révélé par la CCM. Le produit est purifié après évaporation par chromatographie sur colonne de silice. On obtient le dérivé du titre sous forme d'un produit blanc amorphe présentant un seul spot de Rf 0,70 dans un système chloroforme 98 acétone 2 - RMN dans $CDCl_3$/TMS : 7,7 et 7,4 ppm 2 (m) 4H indène ; 7,3 ppm (s) 5H phényl ; 5,0 ppm (1) 1H en 2 ; 4,60 ppm (s) 4H, 2 x O<u>$CH_2$</u>CO ; 4,1 ppm (q) 4H 2 x <u>$CH_2$</u>$CH_3$ ; 1,15 ppm (t) 6H 2 x $OCH_2$<u>$CH_3$</u>.

<u>EXEMPLE 31 - BIS (HYDROXYCARBONYLMETHLYOXYIMINO) -1,3 PHENYL-2 (1H) INDENE (2H)</u>

a) Bis (terbutoxycarbonylméthoxyimino)-1,3 phényl-2

(1H) indène (2H).

On opère dans les conditions de l'exemple précédent en utilisant les quantités équivalentes de tertbutoxy-carbonylméthyloxyamine chlorhydrate (voir exemple 11).

b) Bis (hydroxycarbonylméthyloxy amino)-1,3 phényl-2 (1H) indène (2H) -

Le dérivé obtenu au paragraphe a) est traité suivant la technique décrite à l'exemple 13. On obtient le dérivé du titre sous forme de cristaux blancs de pF > 200°C (déc) et dont on peut réaliser les sels de bases minérales solubles ainsi que les sels d'éthanol-amine et de meglumine.

EXEMPLE 32 - BIS (DIETHYLAMINO-2 ETHYLOXYIMINE)-5,7-PHENYL-6 DIHYDRO-6,7 (5H) DIBENZO (a,c) CYCLOHEPTENE, DICHLORHYDRATE

A 250 ml d'éthanol contenant 30 g (0,1 M) de dioxo-5,7 - phényl-6 dihydro-6,7 -(5H)- dibenzo [a,c] cycloheptène préparé selon B. Aleksiev (Chem. Bericht. 100, 701 [1967], on ajoute 200 mMoles de chlorhydrate de diéthylaminoéthoxyamine. On porte à reflux 24 heures jusqu'à disparition des produits de départ puis traite de façon habituelle. Après purification de la base brute sur colonne d'alumine par le chloroforme et conversion en chlorhydrate dans l'éther chlorhydrique, on aboutit au dérivé du titre sous forme de cristaux blancs de pF =165°C présentant en CCM sur silice dans le système B une tache légèrement dédoublée. La RMN de la base en solution dans $CDCl_3$ donne les signaux suivants : 7,4 ppm (m) 8H, arom ; 7,25 ppm (s) large 5H phényl ; 4,5 ppm (m) 4H,2x = $N-OCH_2-$ ; 4,25 ppm (s) 1H, CH en 6 ; 3,2 à 2,7 ppm (m) 12H, 6 x $-N-\underline{CH_2}$ ; 1,2 ppm (t) 12H 4 x $- CH_2 - \underline{CH_3}$.

En utilisant les conditions de l'exemple précédent avec des dioxo-5,7 phényl-6 dihydro-6,7 - (5H) -dibenzo (a,c) cycloheptène diversement substitués sur le noyau

phényl-6, on obtient les différents produits suivants :

EXEMPLE 33 - BIS (DIETHYLAMINO-2 ETHOXYIMINO) - 5,7 (FLUORO-4) PHENYL-6 DIHYDRO-6,7 - (5H) DIBENZO (a,c) CYCLOHEPTENE, DICHLORHYDRATE pF = 176°C

EXEMPLE 34 - BIS (DIETHYLAMINO-2ETHOXYIMINO) - 5,7 (CHLORO-4) PHENYL-6 DIHYDRO-6,7 - (5H) DIBENZO (a,c) CYCLOHEPTENE, DICHLORHYDRATE pF = 185°C

EXEMPLE 35 - BIS (DIETHYLAMINOETHOXYIMINO) - 5,7 (METHOXY-4) PHENYL-6 DIHYDRO-6,7-(5H) DIBENZO (a,c) CYCLOHEPTENE, DICHLORHYDRATE pF = 155°C

EXEMPLE 36 - BIS (DIETHYLAMINOETHOXYIMINO)-5,7 (METHYL-4) PHENYL-6 DIHYDRO-6,7-(5H) DIBENZO (a,c) CYCLOHEPTENE, DICHLORHYDRATE pF = 170°C

EXEMPLE 37 - ETUDE DES EFFETS SUR L'AGREGATION PLAQUETTAIRE (PLASMA HUMAIN ENRICHI EN PLAQUETTES) INDUITE IN VITRO PAR : ADP, COLLAGENE, ACIDE ARACHIDONIQUE

Les prélèvements sanguins de donneurs volontaires sont traités et ajustés à une concentration plaquettaires de 300 000/cm², puis incubés avec différentes concentrations des produits à tester. Les agrégations sont induites par 2,0 µg/mL d'ADP, par 10,0 µg/mL de collagène.

Les mesures sont faites en utilisant un agrégomètre de type Bryston - ou CHRONOLOG - les changements de densité optique sont notés et les valeurs des concentrations entraînant une inhibition de 50 % sont calculées et reportées dans le tableau suivant :

| DERIVE | $IC_{50}$ en µg/mL | |
|---|---|---|
| | Inducteur : ADP | Inducteur : collagène |
| Ex. 1 | 650 | 800 |
| 2 | - | 800 |
| 3 | 620 | 500 |
| 4 | 600 | 420 |

| | | |
|---|---|---|
| 5 | 420 | 650 |
| 6 | 460 | 600 |
| 7 | 600 | 600 |
| 8 | 650 | 800 |
| 9 | 600 | 600 |
| 10 | 400 | 650 |
| 11 | 200 | 320 |
| 12 | 450 | 670 |
| 13 | 400 | 450 |
| 14 | 460 | 600 |
| 15 | 10 | 24 |
| 16 | 16 | 22 |
| 17 | 10 | 16 |
| 18 | 40 | 50 |
| 19 | 100 | 175 |
| 20 | 80 | 120 |
| 21 | 24 | 40 |
| 22 | 160 | 145 |
| 23 | 420 | 350 |
| 24 | 210 | 340 |
| 25 | 20 | 38 |
| 26 | 18 | 24 |
| 27 | 28 | 40 |
| 28 | 40 | 62 |
| 29 | 100 | 110 |
| 30 | 210 | 150 |
| 31 | - | 250 |
| 32 | 18 | 20 |
| 33 | 22 | 12 |
| 34 | 24 | 40 |
| 35 | 16 | 25 |

REVENDICATIONS

1 - Dérivés Bêta bis (alkyloxyimino) cycloalcènes à doubles liaisons conjugées répondant à la formule générale suivante :

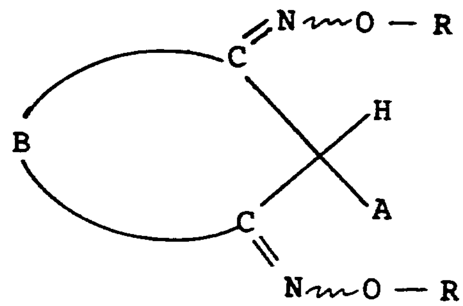

(I)

dans laquelle ;

- A représente l'hydrogène, un groupe phényle, ou un groupe phényle substitué par ;
  - un ou deux atomes d'halogène comme le fluor, le chlore, le brome, l'iode,
  - un ou deux radicaux alkyle inférieur qui peuvent être un groupement méthyle, éthyle, propyle, isopropyle, cyclopropyle, n-butyle, sec-butyle, tertio-butyle,
  - un ou deux radicaux alkoxy choisi parmi les groupes méthoxy, éthoxy, propoxy, isopropoxy, butoxy, tertiobutoxy
  - un ou des substituants choisis parmi les groupes ; nitro, cyano, diméthylamino, diéthylamino,
  - plusieurs substituants choisis dans chacun des différents groupes ci-dessus,
  - un groupement o-méthylènedioxy, o-éthylène dioxy,
- R représente :
- un radical alkyle saturé ou insaturé, linéaire ou ramifié comprenant de 1 à 16 atomes de carbone,
- un radical cycloalkylalkyle ou cycloalkyle comprenant au total de 4 à 16 atomes de carbone et dont le cycle comprend de 3 à 8 atomes de carbone,
- un radical phényle ou phénylalkyle, le motif alkyle comprenant de 1 à 3 enchaînements méthylènes,
- un radical alkyle linéaire ou ramifié comprenant de 1 à 6 atomes de carbone substitué par un groupe $-N\ R_1R_2$, où $R_1$

et $R_2$ identiques ou différents sont des atomes d'hydrogène, des groupes alkyles linéaires ou ramifiés comprenant de 1 à 4 atomes de carbone éventuellement substitués par un groupe hydroxy, méthoxy, éthoxy, ou bien $R_1$ et $R_2$ identiques ou différents peuvent déterminer un hétérocycle azoté saturé ou insaturé, ou un hétérocycle saturé ou insaturé comprenant un atome d'azote et un hétéroatome qui peut être le soufre, l'oxygène ou l'azote, éventuellement substitué,

- un hétérocycle comprenant de 5 à 7 chaînons et comprenant 1 ou 2 hétéroatomes choisis parmi l'azote, le soufre, l'oxygène, relié par un enchaînement méthylène comprenant de 1 à 4 atomes de carbone,
- un radical alkyle linéaire ou ramifié saturé ou insaturé comprenant de 1 à 16 atomes de carbone et substitué par :
  . un groupement CN ou bien
  . un groupement -COOQ, Q étant l'hydrogène, un radical alkyle linéaire ou ramifié comprenant de 1 à 6 atomes de carbone, un radical phénylméthyle, phényle, ces cycles phényles étant éventuellement substitués par un halogène, un groupe cyano, un groupe méthyle, un groupe méthoxy ou un groupe trifluorométhyle.
- B représente l'enchaînement :

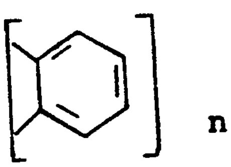

n étant égal à 1 ou 2,
- une double liaison d'un noyau benzénique condensé,
- deux doubles liaisons conjuguées entre elles et avec les groupes imino, comprises chacune dans un noyau benzénique condensé,

et leurs sels pharmacologiquement acceptables lorsque R contient un groupe salifiable.

2. Dérivé selon la Revendication 1, caractérisé en ce

qu'il est constitué par un mélange des isomères ZZ-ZE-EE.

3. Dérivé selon la Revendication 1, caractérisé en ce qu'il est constitué par un isomère pur ZZ ou ZE ou EE.

4. Dérivés selon les Revendications 1 à 3, caractérisés en ce que R représente un radical alkyle-COOQ, Q étant l'hydrogène ou un alkyle de $C_1$ à $C_6$.

5 - Dérivés selon les revendications 1 à 3 caractérisés en ce que R représente un hétérocycle à cinq ou six chaînons comprenant un ou deux hétéroatomes pouvant être l'azote, le soufre, l'oxygène, liés à un groupement méthylène ou éthylène.

6 - Dérivés selon les revendications 1 à 3 caractérisés en ce que R représente un radical diéthylaminoalkyle, le groupement alkyle comprenant de 2 à 6 atomes de carbone.

7 - Dérivés selon les revendications 1 à 3 et 6, caractérisés en ce que R représente le groupement diéthylaminoéthyle.

8 - Dérivés selon les revendications 1 à 3 où $R_1$ et $R_2$ représentent chacun un atome d'hydrogène.

9 - Dérivés selon les revendications 1 à 3 où A représente un phényle substitué en 4.

10 - Dérivés selon les revendications 1 à 3 où A représente un phényle substitué en 4 par un halogène.

11 - Dérivés selon les revendications 1 à 3 où A représente un phényle non substitué.

12 - Dérivés selon les revendications 1 à 3 où A est l'hydrogène.

13 - Dérivés selon les revendications 1 à 3 et 5, où $R_1R_2$ déterminent avec l'atome d'azote un hétérocycle à 6 chaînons comprenant un ou deux atomes d'azote.

14 - Dérivés selon les revendications 1 à 3,6 et 7, dans lesquelles A est un phényle non substitué.

15 - La Bis (O-diéthylaminoéthyl)oxime du dioxo-5, 7 dihydro-6,7 (5H) dibenzo [a,c] cycloheptène.

16 - Procédé de préparation des dérivés selon la

revendication 1, caractérisé en ce que l'on fait réagir le ß dicéto cycloalcène correspondant avec l'halogéno-hydrate d'une hydroxylamine O-substitué $H_2N-OR$, où R a la même signification que dans la Revendication 1, dans la pyridine et/ou un alcool comprenant de 1 à 3 atomes de carbone, en présence éventuellement d'une base, et en ce que l'on procède éventuellement à une déprotection du produit ainsi obtenu dans le cas où le groupe R contient un groupe protecteur.

17 - Procédé selon la revendication 16, caractérisé en ce que la déprotection s'effectue par hydrolyse basique d'un ester pour conduire à un acide.

18 - Procédé selon la revendication 16, caractérisé en ce que la déprotection s'effectue par hydrogénolyse d'un groupe protecteur benzyloxycarbonyle pour conduire à une amine.

19 - Procédé selon la revendication 16, caractérisé en ce que la déprotection s'effectue par hydrogénolyse d'un ester benzylique pour conduire à un acide.

20 - Médicaments caractérisés en ce qu'ils contiennent au moins un des dérivés selon la revendication 1.

0236151

Office européen des brevets

# RAPPORT DE RECHERCHE EUROPEENNE

Numéro de la demande

EP 87 40 0069

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.4) |
| A,D | EP-A-0 112 584 (GÖDECKE)<br>* Revendications 1,5 *<br>--- | 1,14 | C 07 C 131/08<br>C 07 D 213/30<br>A 61 K 31/15<br>A 61 K 31/44 |
| A,D | CHEMISCHE BERICHTE, vol. 104, 1971, pages 1573-1579, Verlag Chemie GmbH, Weinheim/Bergstr., DE; W. RIED et al.: "Synthese und Reaktionen des 5.7-Dioxo-6.7-dihydro-5H-dibenzo[a.c]cycloheptens"<br>* Page 1574, lignes 14-16 *<br>--- | 1 | |
| A | GB-A-1 153 431 (COLGATE-PALMOLIVE)<br>* Revendications 1,14 * & US-A-3 349 128 (Cat. D)<br>----- | 1,14 | |
| | | | DOMAINES TECHNIQUES RECHERCHES (Int. Cl.4) |
| | | | C 07 C 131/00<br>C 07 D 213/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 28-04-1987 | BRENNAN J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82